# EUROPEAN PATENT SPECIFICATION

(11) **EP 3 703 635 B1**
(45) Date of publication and mention of the grant of the patent: **23.07.2025**
(21) Application number: 18808112.9
(22) Date of filing: 30.10.2018
(51) Int. Cl.: A61F 13/00, A61F 13/02

(54) **NEGATIVE PRESSURE WOUND THERAPY ARTICLE**
ARTIKEL FÜR UNTERDRUCKWUNDTHERAPIE
ARTICLE DE TRAITEMENT DE PLAIE PAR PRESSION NÉGATIVE

(30) Priority: 31.10.2017 US 201762579476 P; 14.12.2017 US 201762598697 P
(43) Date of publication of application: 09.09.2020
(73) Proprietor: Solventum Intellectual Properties Company, Maplewood, MN 55144 (US)
(72) Inventor: FITZSIMONS, Robert T., Jr., Saint Paul, Minnesota 55133-3427 (US); BAKER, Bryan A., Saint Paul, Minnesota 55133-3427 (US); NINKOVIC, Jana, Saint Paul, Minnesota 55133-3427 (US); LIU, Jie, Saint Paul, Minnesota 55133-3427 (US); SGOLASTRA, Federica, Saint Paul, Minnesota 55133-3427 (US); DAI, Minghua, Saint Paul, Minnesota 55133-3427 (US); ZHANG, Wei, Saint Paul, Minnesota 55133-3427 (US); MCNULTY, Amy K., Saint Paul, Minnesota 55133-3427 (US)
(74) Representative: Mathys & Squire
(86) International application number: PCT/IB2018/058493
(87) International publication number: WO 2019/087065

(56) References cited:
- EP-A1- 2 415 426
- WO-A1-2015/179235
- WO-A1-2018/005275
- US-A1- 2011 077 605
- US-A1- 2017 007 462

## Description

### BACKGROUND

Clinical studies and practice have shown that providing a negative pressure in proximity to a tissue site promotes the growth of new tissues at the tissue site. The application of negative pressure is successful in treating wounds. This treatment (frequently referred to in the medical community as "negative pressure wound therapy (NPWT)," "reduced pressure therapy," or "vacuum therapy") provides a number of benefits, including faster healing and increased formulation of granulation tissue. Typically, reduced pressure is applied to tissues through a foam, a pad or other manifolding device, such as gauze. The manifolding device typically contains cells, pores or other openings that are capable of distributing reduced pressure to the tissue and channeling fluids that are drawn from the tissue. The porous pad often is incorporated into a dressing having other components that facilitate treatment.

US 2017/007462 A1 discloses an apparatus for treating a wound with negative pressure therapy and a method of manufacturing the apparatus. The apparatus comprises a bespoke wound filler. The bespoke wound filler may incorporate a stabilizing structure. In one example, the stabilizing structure is comprised of two or more interlocking strips. A bottom strip and a top strip may be used to make the stabilizing structure. Each of the top and bottom strips are preferably configured to movably interlock with each other, for example via matching notches. Each individual strip may be formed as a "wavy" strip (when seen from a vertical orientation) that, when joined face-to-face, form one or more circular or ovoid cells.

EP 2 415 426 A1 discloses a wound packing for use with suction. The wound packing can comprise a corrugated unit comprising two sheets, a first sheet layer and a second sheet-like layer having an essentially sinusoidal cross section, for example, that is coupled to the surface of the first sheet layer at locations. The corrugated unit can also be used to form more complex three-dimensional structures, such as a spiral wound packing that is formed by rolling the corrugated unit to expose a portion of the first sheet layer along the circumference.

US 2011/077605 A1 discloses a wound packing that includes polyester fibers or comparable resilient fibers in a corrugated layer pattern configured to form a resilient compressible structure.

WO 2018/005275 A1 discloses a negative pressure wound therapy article comprising a network of interconnected polymeric strands. Each of the interconnected polymeric strands has a first surface adapted to contact a tissue site. At least one of the interconnected polymeric strands has a plurality of features extending from the first surface of the interconnected polymeric strands. At least one of the interconnected polymeric strands is non-linear. The negative pressure wound therapy article further comprises a plurality of openings between adjacent interconnected polymeric strands.

WO 2015/179235 A1 discloses a discontinuous silicone article that includes a plurality of strands of radiation cured silicone gel arranged to form a net-like structure with openings between strands. The silicone gel provides adhesion to a surface, such as skin, and the openings provide for moisture transmission away from the surface.

Other known NPWT articles are discussed in U.S. Patent Nos. 7,494,482; 8,057,447; 8,889,243 and 9,107,989.

### SUMMARY

In one aspect, the present disclosure provides an article comprising: a network of interconnected polymeric strands; wherein each of the interconnected polymeric strands has a height (H1) of from 100 to 2000 µm and an average width (T) of from 10 to 500 µm; wherein each of the interconnected polymeric strands has a first surface adapted to contact a tissue site; wherein none of the interconnected polymeric strand has a feature extending from the first surface of the interconnected polymeric strands; wherein at least one of the interconnected polymeric strands is non-linear; and a plurality of openings between adjacent interconnected polymeric strands; wherein the article is a negative pressure wound therapy article; and wherein the aspect ratio of the openings is in the range of from greater than 1:1 to 100:1, wherein the aspect ratio is a length (L1) to width (W1) ratio of the openings, wherein the length (L1) of an opening is the longest lateral distance parallel to x direction, and the width (W1) of an opening is the longest distance parallel to y direction.

In another aspect, the present disclosure provides a system including the article of present invention and a reduced pressure source fluidly connected to the opening of the article to deliver the reduced pressure through the opening, and to the tissue site.

Various aspects and advantages of exemplary embodiments of the present disclosure have been summarized. The above Summary is not intended to describe each illustrated embodiment or every implementation of the present disclosure. Further features and advantages are disclosed in the embodiments that follow. The Drawings and the Detailed Description that follow more particularly exemplify certain embodiments using the principles disclosed herein.

### BRIEF DESCRIPTION OF THE DRAWINGS

The disclosure may be more completely understood in consideration of the following detailed description of various embodiments of the disclosure in connection with the accompanying figures, in which:
FIG. 1 illustrates an article according to an embodiment of the present invention.
FIG. 2 illustrates a top view of an article according to an embodiment of the present invention.
FIG. 3 illustrates a schematic cross-section side view of an article according to an embodiment of the present invention.
FIG. 4 illustrates a reduced pressure treatment system according to an embodiment of the present invention.

While the above-identified drawings, which may not be drawn to scale, set forth various embodiments of the present disclosure, other embodiments are also contemplated, as noted in the Detailed Description. In all cases, this disclosure describes the presently disclosed invention by way of representation of exemplary embodiments and not by express limitations. It should be understood that numerous other modifications and embodiments can be devised by those skilled in the art, which fall within the scope of the present invention.

### DETAILED DESCRIPTION

Before any embodiments of the present disclosure are explained in detail, it is understood that the invention is not limited in its application to the details of use, construction, and the arrangement of components set forth in the following description. The invention is capable of other embodiments and of being practiced or of being carried out in various ways that will become apparent to a person of ordinary skill in the art upon reading the present disclosure. Also, it is understood that the phraseology and terminology used herein is for the purpose of description and should not be regarded as limiting. The use of "including," "comprising," or "having" and variations thereof herein is meant to encompass the items listed thereafter as well as additional items. It is understood that other embodiments may be utilized and structural or logical changes may be made without departing from the scope of the present disclosure.

As used in this Specification, the recitation of numerical ranges by endpoints includes all numbers subsumed within that range (e.g. 1 to 5 includes 1, 1.5, 2, 2.75, 3, 3.8, 4, and 5, and the like).

Unless otherwise indicated, all numbers expressing quantities or ingredients, measurement of properties and so forth used in the Specification and embodiments are to be understood as being modified in all instances by the term "about." Accordingly, unless indicated to the contrary, the numerical parameters set forth in the foregoing specification and attached listing of embodiments can vary depending upon the desired properties sought to be obtained by those skilled in the art utilizing the teachings of the present disclosure. At the very least, and not as an attempt to limit the application of the doctrine of equivalents to the scope of the claimed embodiments, each numerical parameter should at least be construed in light of the number of reported significant digits and by applying ordinary rounding techniques.

The article of the present application is well suited to promote tissue growth at the tissue site yet prevent in-growth of new tissue into the article. The article of the present application can help to deliver a significant portion of microstrain to the wound site by the architecture of the article, for example, the surface morphology of the article and thus may allow for lower pressure settings for NPWT to be used (for example, -75 mmHg vs -125 mmHg). This may allow a longer battery life of the NPWT system and the use of smaller pumps for the NPWT.

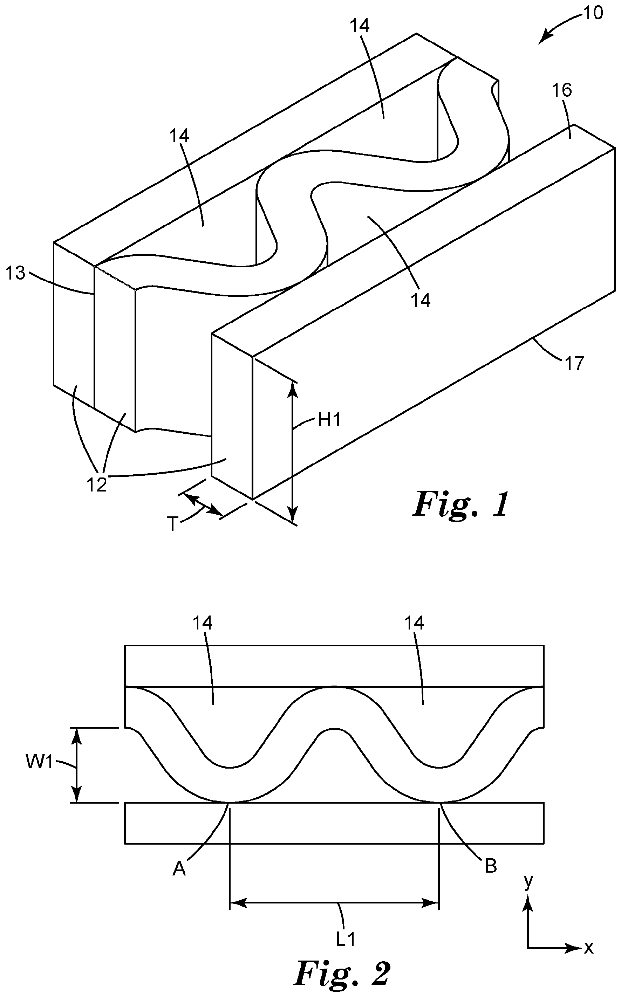

Referring to FIG. 1, a NPWT article according to an embodiment of the present invention includes a network of interconnected polymeric strands, or sheets 12 and a plurality of openings 14 between adjacent polymeric strands. Polymeric strands 12 can be connected at connections 13. Typically, there are a plurality of connections 13 between adjacent strands. Polymeric strands 12 have a tissue contact surface 16 as a first surface and a second surface 17 opposite the first surface. The first surface 16 does not have a feature, or a protrusion that extend from the first surface 16. In other words, none of the interconnected polymeric strand has a feature extending from the first surface of the interconnected polymeric strands. The openings 14 form or provide open fluid channels from the first surface 16 of the network of polymeric strands to a second surface opposite first surface 16. Through the open fluid channels, openings 14 are typically used to allow reduced pressure applied to a tissue site.

Referring more specifically to FIG. 1, the height, H1, of each polymeric strand 12 is from 100 to to 2000 micrometers, and may be up to 1500 micrometers, up to 1000 micrometers, up to 500 micrometers, or up to 400 micrometers. In some embodiments, the height, H1, of each polymeric strand 12 may be no less than 200 micrometers, or no less than 300 micrometers. In some embodiments, the height, H1, of each polymeric strand 12 may be between 200 and 1500 micrometers, between 300 and 1000 micrometers, between 300 and 500 micrometers or between 300 and 400 micrometers. The thickness, T, of each polymeric strand 12 has an average width of from 10 to 500 micrometers, and in some embodiments up to 400 micrometers, or up to 250 micrometers. In some embodiments, the thickness, T, of each polymeric strand 12 may have an average width in a range from 10 micrometers to 400 micrometers, or 10 micrometers to 250 micrometers. In some embodiments, the article comprising interconnected polymeric strands has an average thickness not greater than 5 mm. In one embodiment of the present invention, the height and thickness of the interconnected polymeric strands 12 is uniform for a particular article 10. In other embodiments, the height and thickness of the interconnected polymeric strands 12 may be different. For example, the interconnected polymeric strands 12 having different height. Similarly, thickness of the interconnected polymeric strands 12 may vary. In some, embodiments, the interconnected polymeric strands 12 may have a range of thicknesses, for example, the interconnected polymeric strands 12 tends to be thinnest where it abuts an opening.

In some embodiments, the article comprising interconnected polymeric strands has a thickness up to 2 mm, up to 1 mm, up to 500 micrometers, up to 250 micrometers, up to 100 micrometers, up to 75 micrometers, up to 50 micrometers, or up to 25 micrometers. In some embodiments, the article comprising interconnected polymeric strands has a thickness no less than 10 micrometers. In some embodiments, the article comprising interconnected polymeric strands has a thickness in a range from 10 micrometers to 2 mm, 10 micrometers to 1 mm, 10 micrometers to 750 micrometers, 10 micrometers to 500 micrometers, 10 micrometers to 250 micrometers, 10 micrometers to 100 micrometers, 10 micrometers to 75 micrometers, 10 micrometers to 50 micrometers, or 10 micrometers to 25 micrometers. In some embodiments, the article comprising interconnected polymeric strands has an average thickness in a range from 250 micrometers to 5 mm.

In some embodiments, at least one of the interconnected polymeric strands 12 may be non-linear. In some embodiments, at least 25% of the interconnected polymeric strands 12 may be non-linear. In some embodiments, at least 50% of the interconnected polymeric strands 12 may be non-linear. In some embodiments, at least 75% of the interconnected polymeric strands 12 may be non-linear. In some embodiments, essentially all (more than 90%, but less than 100%) the interconnected polymeric strands 12 may be non-linear. In some embodiments, all of the interconnected polymeric strands 12 may be non-linear. In some embodiments, the non-linear polymeric strand may have a shape of a curve. In some embodiments, the non-linear polymeric strand may have a shape of a sinusoidal curve. In some embodiments, the non-linear polymeric strand may have a shape of a sinusoidal curve. In other embodiments, at least one of the interconnected polymeric strands 12 may be linear. In some other embodiments, 25% to 75% of the interconnected polymeric strands 12 may be linear. In some other embodiments, 50% to 75% of the interconnected polymeric strands 12 may be linear. In certain embodiments, the network of interconnected polymeric strands may include alternating non-linear polymeric strands and linear polymeric strands, as shown in FIG. 2. In some embodiments, the interconnected polymeric strands 12 are oriented in the same direction, for example, x direction as illustrated in FIG. 2. In some embodiments, the interconnected polymeric strands 12 do not substantially cross over each other (i.e., at least 50 (at least 55, 60, 65, 70, 75, 80, 85, 90, 95, 99, or even 100) percent by number do not cross over each other).

The aspect ratio (a ratio of the length to the width) of the openings 14 is in a range of from greater than 1:1 to 100:1 and may be greater than 1.5:1, 2:1, 3:1 or 5:1. In some embodiments, the aspect ratio (a ratio of the length to the width) of the openings 14 may be in a range from 1:1 to 75:1, 1:1 to 50:1, 1:1 to 25:1, 2:1 to 100:1 2:1 to 75:1, 2:1 to 50:1, 2:1 to 25:1, or 2:1 to 10:1. The length, L1, of an opening 14 illustrated in FIG. 2 is the longest lateral distance parallel to x direction, for example, the length between connections A and C. If the non-linear polymeric strand has a shape of a sinusoidal curve, the length of the opening 14 equals the wavelength of the sinusoidal curve. The width, W1, of an opening 14 illustrated in FIG. 2 is the longest distance parallel to y direction. If the non-linear polymeric strand has a shape of a sinusoidal curve, the width of the opening 14 equals two times amplification of the sinusoidal curve. Openings 14 of the article may have a range of L1 and W1 values as a result in part of variable spacing of the connections A and B. In some embodiments, the openings have widths, W1, up to 10 mm, up to 1 mm or up to 0.5 mm. In some embodiments, the openings have widths, W1, at least 5 micrometers or at least 10 micrometers. In some embodiments, the openings have widths, W1, in a range from 5 micrometers to 1 mm or from 10 micrometers to 0.5 mm. In some embodiments, the openings have lengths, L1, up to 10 mm or up to 1 mm. In some embodiments, the openings have lengths, L1, at least 100 micrometers. In some embodiments, the openings have lengths, L1, in a range from 100 micrometers to 10 mm or from 100 micrometers to 1 mm. FIGs 1 and 2 are idealized illustrations of one embodiment of the present application. In some embodiments, the openings 14 may have irregularly formed perimeters. This can mean that the openings have irregular shapes (that is, no lines of symmetry). They may have edges that are not smooth (e.g., jagged or feathery edges). Irregularly formed openings can also have a variety of thicknesses of the polymeric strands surrounding the openings.

In some embodiments, openings 14 may have any suitable shape, for example, a shape selected from shapes of ellipse, oval, pointed oval (or lens), diamond, ½ ellipse, ½ oval, ½ lens, triangle, etc. In some embodiments, the openings of the mechanical fastening nets described herein have at least two pointed ends. In some embodiments, at least some of the openings are elongated with two pointed ends. In some embodiments, at least some of the openings are elongated with two opposed pointed ends. In some embodiments, at least some of the openings are ovals.

In some embodiments, the article described herein have a total open area for each of the first and second, generally opposed surfaces of not greater than 50 (in some embodiments, not greater than 45, 40, 35, 30, 25, 20. 15, 10, 5, 4 3, 2, 1, 0.75, 0.5, 0.25, or even not greater than 0.1) percent of the total area of the respective surface. In some embodiments, for at least a majority of the openings of the article described herein, the maximum area of each opening is not greater than is 5 (in some embodiments, not greater than 2.5, 2, 1, 0.5, 0.1, 0.05, 0.01, 0.075, or even not greater than 0.005)mm². Individual openings range from 0.005 mm² to 5 mm². In some embodiments, the article according to the present disclosure have in a range from 50,000 to 6,000,000 (in some embodiments, 100,000 to 6,000,000, 500,000 to, 6,000,000, or even 1,000,000 to 6,000,000) openings/m².

In some embodiments, the tensile strength of the article parallel to x direction is greater than the tensile strength of the article parallel to y direction. Therefore, the article is easier to be stretched in y direction than in x direction. In some embodiments, the tensile strength of the article parallel to x direction is at least 2.23 MPa, at least 2.25 MPa, at least 2.5 MPa or at least 3.0 MPa. In some embodiments, the tensile strength of the article parallel to x direction is up to 5.42 MPa, up to 5.3 MPa, up to 5.0 MPa, or up to 4.5 MPa. In some embodiments, the tensile strength of the article parallel to x direction is from 2.23 MPa to 5.42 MPa., from 2.5 MPa to 5.0 MPa or from 3.0 MPa to 4.5 MPa. The Young's modulus of the article is up to 10.6 MPa, up to 10.0 MPa, up to 9.0 MPa, or up to 8.0 MPa. The Young's modulus of the article is at least 3.85 MPa, at least 4.0 MPa has a range from 3.85 MPa to 10.6 MPa in a direction parallel to x direction.

The presence and sizing of the opening allow opening to distribute reduced pressure to the tissue site. In addition to distributing reduced pressure to the tissue site, the article 10 also serves to impart stresses and strains to the tissue site similar to those seen with cellular foam that traditionally has been used in reduced pressure systems. Other materials sometimes used in reduced pressure systems, such as gauze, do not have this effect on tissue. Unbound by the theory, the stresses and strains created by the article 10 are believed to cause micro-deformation of existing tissues and plays a significant role in the generation of new tissues at the tissue site. The amount of stress and strain imparted to a tissue site is determined by the amount of reduced pressure supplied to the tissue site and the surface morphology of the article that contacts the tissue site. As reduced pressure is applied, portions of the tissue site are pulled against the article 10, which results in the development of stresses and strains within the tissue. In some embodiments, the article of the present disclosure can be a mechanical fastening net or a mechanical fastening sheet.

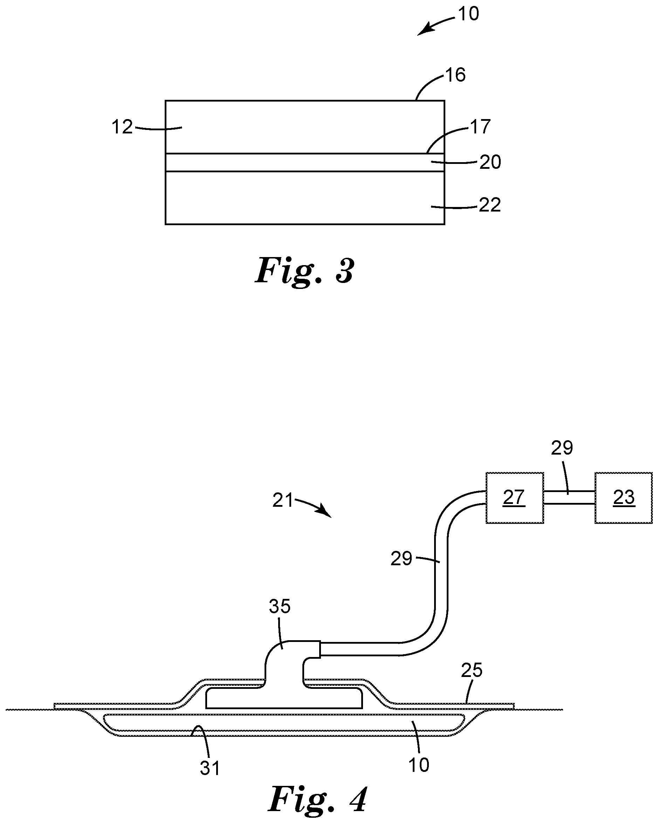

Referring to FIG. 3, in some embodiments, the article 10 may further include an adhesive layer 20 in contact with the second surface 17 of the interconnected polymeric strands 12. Suitable adhesive for use in the adhesive layer 20 of the article 10 can include any adhesive that provides acceptable adhesion to skin and is acceptable for use on skin (e.g., the adhesive should preferably be non-irritating and non-sensitizing). Suitable adhesives can be pressure sensitive and in certain embodiments have a relatively high moisture vapor transmission rate to allow for moisture evaporation. Suitable pressure sensitive adhesives include those based on acrylates, urethane, hyrdogels, hydrocolloids, block copolymers, silicones, rubber based adhesives (including natural rubber, polyisoprene, polyisobutylene, butyl rubber etc.) as well as combinations of these adhesives. The adhesive component may contain tackifiers, plasticizers, rheology modifiers as well as active components including for example an antimicrobial agent. Suitable adhesive can include those described in U.S. Patent Nos. 3,389,827; 4,112,213; 4,310,509; 4,323,557; 4,595,001; 4,737,410; 6,994,904 and International Publication Nos. WO 2010/056541; WO 2010/056543 and WO 2014/149718.

The article 10 may further include a cellular foam or another material 22 in contact with the adhesive layer, the adhesive layer in between the network of interconnected polymeric strands and the cellular foam or another material 22. In some other embodiments, the cellular foam or another material 22 that is positioned adjacent to or attached to the surface 17 of the interconnected polymeric strands opposite the features 18. The use of a cellular foam or other material 22 increases the ability of the reduced pressure conduit 29 or the distribution adapter 35 to deliver and distribute reduced pressure to the article 10. The features 18 and interconnected polymeric strands serve as a barrier to new tissue growth entering pores of the cellular foam or other material.

Referring to FIG. 4, a reduced pressure treatment system 21 according to an embodiment of the present invention includes a reduced pressure dressing, or article 10 fluidly connected to a reduced pressure conduit 29. The reduced pressure conduit 29 is fluidly connected to a reduced pressure source 23 such as a vacuum pump or another source of suction. The article 10 is placed against a tissue site 31 of a patient and is used to distribute a reduced pressure provided by the reduced pressure source 23. Typically, reduced pressure is maintained at the tissue site by placing an impermeable or semi-permeable cover 25 over the article 10 and the tissue site 31. The reduced pressure also serves to draw wound exudates and other fluids from the tissue site 31. A canister 27 may be fluidly connected to the reduced pressure conduit 29 and disposed between the article 10 and the reduced pressure source 23 to collect the fluids drawn from the tissue site 31. A distribution adapter 35 may be connected to the reduced pressure conduit 29 and positioned on the article 10 to aid in distributing the reduced pressure to the article 10.

In some embodiments, the interconnected polymeric strands 12 can include an elastomeric polymer. Elastomeric polymer can be any suitable elastomeric polymer, including but not limited to polyolefins and polyurethanes. In some embodiments, elastomeric polymer can be a medical grade material that is relatively impermeable to fluid flow. Alternatively, elastomeric polymer can be a semi-permeable material that allows select fluids or amounts of fluids to pass. In some embodiments, the composition of interconnected polymeric strands 12 may be formed from different materials.

Wound-treatment methods using articles of the present invention can include positioning the article on a wound of a patient and applying a reduced pressure to the wound through the article (e.g., through the openings). Such methods can further comprise: coupling a drape to skin adjacent the wound such that the drape covers the article and the wound, and forms a space between the drape and the wound. In some cases, positioning the article on the wound can include placing the article over the wound on the first surface facing the wound. In some cases, applying the reduced pressure to the wound comprises activating a vacuum source (e.g., reduced pressure source 23 of FIG. 4) that is coupled to the article. Some cases may comprise: delivering a fluid to the wound through the article. In some embodiments, delivering a fluid comprises activating a fluid source that is coupled to the article.

The following working examples are intended to be illustrative of the present disclosure and not limiting.

### EXAMPLES

All animal studies were conducted with approval of the internal Institutional Animal Care and Use Committee (IACUC) in compliance with the United States Guide for the Care and Use of Laboratory Animals and the United States Animal Welfare Act (9CFR).

### Example 1.

A polyolefin net was prepared using ENGAGE 8200 polyolefin elastomer (obtained from the Dow Chemical Company, Midland, MI) according to the methods described in United States Patent Application 2014/0234606 (Ausen). The resulting net material had strand thickness (T) ranges of about 0.38-0.83 mm, opening width (W1) ranges of about 0.15-0.57 mm, opening length ranges (L1) of about 1.5-2.1 mm, and strand height (H1) ranges of about 0.60-1.24 mm.

### Example 2.

Full thickness wounds (5 cm in diameter, 1 cm deep) were surgically created on the backs of anesthetized Yucatan mini pigs (female, less than 36 weeks old and less than 35 Kg). Each wound was allowed to reach hemostasis and then wiped with gauze. A pad (5 cm diameter, 10 mm thick) of GRANUFOAM polyurethane foam (V.A.C Granufoam Dressing Medium, KCI Incorporated, San Antonio, TX) was placed in the wound. A conduit (3 mm I.D. plastic tubing) was connected at one end to the foam section and the other end was connected to a vacuum pump. The foam pad was covered with an occlusive, adhesive film that was adhered to skin adjacent to the wound. The vacuum pump was engaged with a pressure setting of -125 mm Hg and the foam pad was maintained in place for 3 days. Vacuum was disconnected and the foam pad was removed from the wound. The resulting exposed wound was treated by first placing the net of Example 1 (5 cm diameter section) in the wound. The net was then covered with a fresh pad (5 cm diameter, 10 mm thick) of GRANUFOAM polyurethane foam to form the test dressing. The foam pad section was connected to the vacuum pump and the test dressing was covered with an occlusive, adhesive film that was adhered to skin adjacent to the wound. The vacuum pump was engaged with a pressure setting of -125 mm Hg. The finished test dressings were maintained on the wounds for treatment times of either 3 days or 6 days.

At the end of the designated treatment period, the adhesive film and vacuum conduit were carefully removed and the peel force required to remove the test dressing from the wound was measured. Peel force was determined using a Shimpo digital force gauge (#FGV-100XY from Shimpo Instruments, Glendale Heights, IL) equipped with TORIEMON data acquisition software. The force gauge was mounted onto a motorized stage and positioned such that the initial peel angle was approximately 180 degrees with the retraction speed set at approximately 100 mm/minute. A blunted hook attached to the force gauge was looped through both the foam pad and netting sections to connect the test dressing to the instrument. The peel force was recorded in Newtons (N) as a function of time. The mean Peak Peel Force is reported in Table 1 for the 3 day and 6 day wear of the test dressing.

### Comparative Example 2.

Comparative Example 2 differed from Example 2 in that the dressing applied on the 3^{rd} day after the initial wound creation did not include the net of Example 1. Full thickness wounds (5 cm in diameter, 1 cm deep) were surgically created on the backs of anesthetized Yucatan mini pigs (female, less than 36 weeks old and less than 35 Kg). Each wound was allowed to reach hemostasis and then wiped with gauze. A pad of GRANUFOAM polyurethane foam (5 cm diameter, 10 mm thick) was placed in the wound. A conduit (3 mm I.D. plastic tubing) was connected at one end to the foam section and the other end was connected to a vacuum pump. The foam pad was covered with an occlusive, adhesive film that was adhered to skin adjacent to the wound. The vacuum pump was engaged with a pressure setting of -125 mm Hg and the foam pad was maintained in place for 3 days. Vacuum was disconnected and the foam pad was removed from the wound. The resulting exposed wound was treated by placing a fresh pad of GRANUFOAM polyurethane foam (5 cm diameter, 10 mm thick) in the wound to form the comparative test dressing. The foam pad was connected to the vacuum pump and the comparative test dressing was covered with an occlusive, adhesive film that was adhered to skin adjacent to the wound. The vacuum pump was engaged with a pressure setting of -125 mm Hg. The finished comparative test dressings were maintained on the wounds for treatment times of either 3 days or 6 days.

At the end of the designated treatment period, the adhesive film and vacuum conduit were carefully removed and the peel force required to remove the comparative test dressing from the wound was measured. Peel force was determined using a Shimpo digital force gauge (#FGV-100XY) equipped with TORIEMON data acquisition software. The force gauge was mounted onto a motorized stage and positioned such that the initial peel angle was approximately 180 degrees with the retraction speed set at approximately 100 mm/minute. A blunted hook attached to the force gauge was looped through the foam pad to connect the comparative test dressing to the instrument. The peel force was recorded in Newtons (N) as a function of time. The mean Peak Peel Force is reported in Table 1 for the 3 day and 6 day wear of the comparative test dressing.

**Table 1**

| Example Number | Days of Wear | Mean Peel Force (N) | Number of Samples |
|---|---|---|---|
| Example 2 (Test Dressing) | 3 | 6.0 | 2 |
| Comparative Example 2 (Comparative Test Dressing) | 3 | 7.9 | 3 |
| Example 2 (Test Dressing) | 6 | 4.9 | 2 |
| Comparative Example 2 (Comparative Test Dressing) | 6 | 18.5 | 2 |

The peel test results demonstrate that the test dressings of Example 2 were easier to remove (required less force) than the comparative test dressings of Comparative Example 2. Following removal of the test dressings from the wounds, visual inspection showed that the test dressings of Example 2 had less in-growth of tissue into the foam layer than the comparative test dressings of Comparative Example 2.

### Example 3

A 2.5 cm by 2.5 cm section of the net of Example 1 was prepared and the surface on one side of the net was modified by corona treatment for 15-20 seconds using a hand-held unit with rastering motion (Model BD-20 Laboratory Corona Treater, Electro-Technic Products Company, Chicago, IL). One surface of a 2.5 cm by 2.5 cm (12 mm thick) pad of GRANUFOAM polyurethane foam (V.A.C. Granufoam Dressing Medium, KCI Incorporated, San Antonio, TX) was also modified using the corona treatment procedure described above.

A 2.5 cm by 2.5 cm section of 3M #2477 Double-Coated TPE Silicone/Acrylic adhesive tape (3M Company, Maplewood, MN) that had been perforated (1 mm diameter perforations patterned 3 mm center-to-center) was prepared. The paper release liner was removed and the exposed adhesive surface was heated for 10-20 seconds with hot air from an electric heat gun. The adhesive tape was edge aligned and applied to the corona treated surface of the foam pad. Next, the plastic release liner was removed from the tape and the corona treated surface of the net was edge aligned and applied to the exposed adhesive surface. Hand pressure was applied to the resulting laminate for a few seconds followed by placement of a 0.46 Kg weight on the laminate overnight. The weight was removed to provide the finished laminated article.

### Example 4

A double sided acrylic adhesive transfer tape (3M 300LSE tape #9472LE, 3M Company) was perforated through all layers in a repeating hexagonal pattern with 5 mm diameter perforations spaced 1 cm center-to-center. A 2.5 cm by 2.5 cm section of the net of Example 1 was prepared and the surface on one side of the net was modified by corona treatment for 15-20 seconds using a hand-held unit with rastering motion (Model BD-20 Laboratory Corona Treater). One surface of a 2.5 cm by 2.5 cm (12 mm thick) pad of GRANUFOAM polyurethane foam (V.A.C. Granufoam Dressing Medium) was also modified using the corona treatment procedure described above.

One of the release liners was removed from a 2.5 cm by 2.5 cm section of the double sided adhesive transfer tape and the exposed adhesive surface was heated for 10-20 seconds with hot air from an electric heat gun. The adhesive was edge aligned and applied to the corona treated surface of the foam pad. Next, the second release liner was removed and the corona treated surface of the net was edge aligned and applied to the exposed adhesive surface. Hand pressure was applied to the resulting laminate for a few seconds followed by placement of a 0.46 Kg weight on the laminate overnight. The weight was removed to provide the finished laminated article.

## Claims

1. An article, comprising:
a network of interconnected polymeric strands; wherein each of the interconnected polymeric strands has a height (H1) of from 100 to 2000 µm and an average width (T) of from 10 to 500 µm; wherein each of the interconnected polymeric strands has a first surface adapted to contact a tissue site; wherein none of the interconnected polymeric strand has a feature extending from the first surface of the interconnected polymeric strands; wherein at least one of the interconnected polymeric strands is non-linear; and
a plurality of openings between adjacent interconnected polymeric strands;
wherein the article is a negative pressure wound therapy article, and
wherein the aspect ratio of the openings is in the range of from greater than 1:1 to 100:1, wherein the aspect ratio is a length (L1) to width (W1) ratio of the openings, wherein the length (L1) of an opening is the longest lateral distance parallel to x direction, and the width (W1) of an opening is the longest distance parallel to y direction.

2. The article of claim 1, wherein at least one of the interconnected polymeric strands is linear.

3. The article of any one of claims 1 to 2, wherein the network comprises alternating non-linear polymeric strands and linear polymeric strands.

4. The article of any one of claims 1 to 3, wherein the non-linear polymeric strand has a sinusoidal curve.

5. The article of any one of claims 1 to 4, wherein the tensile strength of the article parallel to x direction is more than 2.23 Mpa.

6. The article of any one of claims 1 to 5, wherein the polymeric strands comprise an elastomeric polymer.

7. The article of claim 6, wherein the elastomeric polymer is selected from polyolefins or polyurethanes.

8. The article of any one of claims 1 to 7, wherein essentially all the interconnected polymeric strands are non-linear.

9. A system, comprising:
the article of any one of claims 1 to 8; and
a reduced pressure source fluidly connected to the opening of the article to deliver the reduced pressure through the opening and to the tissue site.

10. The article of any one of claims 1 to 8 for use for positioning the article on a wound.

11. The article of claim 10, wherein positioning the article on the wound comprises continuously positioning the article on the wound for more than 3 days.

12. The article of claim 10, wherein positioning the article on the wound comprises continuously positioning the article on the wound for more than 4 days.

13. The article of claim 10, wherein positioning the article on the wound comprises continuously positioning the article on the wound for more than 5 days.

## Patentansprüche

1. Ein Erzeugnis, aufweisend:
ein Netzwerk aus miteinander verbundenen Polymersträngen;
wobei jeder der miteinander verbundenen Polymerstränge eine Höhe (H1) von 100 bis 2000 µm und eine durchschnittliche Breite (T) von 10 bis 500 µm aufweist;
wobei jeder der miteinander verbundenen Polymerstränge eine erste Oberfläche aufweist, die geeignet ist, um eine Gewebestelle zu kontaktieren; wobei keiner der miteinander verbundenen Polymerstränge ein Merkmal aufweist, sich das von der ersten Oberfläche der miteinander verbundenen Polymerstränge erstreckt;
wobei mindestens einer der miteinander verbundenen Polymerstränge nichtlinear ist; und
eine Vielzahl von Öffnungen zwischen angrenzenden miteinander verbundenen Polymersträngen;
wobei das Erzeugnis ein Unterdruck-Wundtherapieerzeugnis ist, und
wobei das Seitenverhältnis der Öffnungen im Bereich von größer als 1 : 1 bis 100 : 1 liegt, wobei das Seitenverhältnis ein Verhältnis von Länge (L1) zu Breite (W1) der Öffnungen ist, wobei die Länge (L1) einer Öffnung die längste seitliche Entfernung parallel zu einer x-Richtung ist und die Breite (W1) einer Öffnung die längste Entfernung parallel zu einer y-Richtung ist.

2. Das Erzeugnis nach Anspruch 1, wobei mindestens einer der miteinander verbundenen Polymerstränge linear ist.

3. Das Erzeugnis nach einem der Ansprüche 1 bis 2, wobei das Netzwerk abwechselnd nichtlineare Polymerstränge und lineare Polymerstränge aufweist.

4. Das Erzeugnis nach einem der Ansprüche 1 bis 3, wobei der nichtlineare Polymerstrang eine sinusförmige Kurve aufweist.

5. Das Erzeugnis nach einem der Ansprüche 1 bis 4, wobei die Zugfestigkeit des Erzeugnisses parallel zu der x-Richtung mehr als 2,23 MPa beträgt.

6. Das Erzeugnis nach einem der Ansprüche 1 bis 5, wobei die Polymerstränge ein elastomeres Polymer aufweisen.

7. Das Erzeugnis nach Anspruch 6, wobei das elastomere Polymer aus Polyolefinen oder Polyurethanen ausgewählt ist.

8. Das Erzeugnis nach einem der Ansprüche 1 bis 7, wobei im Wesentlichen sämtliche der miteinander verbundenen Polymerstränge nichtlinear sind.

9. Ein System, aufweisend:
das Erzeugnis nach einem der Ansprüche 1 bis 8; und
eine Unterdruckquelle, die mit der Öffnung des Erzeugnisses in Fluidverbindung steht, um den Unterdruck durch die Öffnung und an die Gewebestelle abzugeben.

10. Das Erzeugnis nach einem der Ansprüche 1 bis 8 zur Verwendung zum Positionieren des Erzeugnisses auf einer Wunde.

11. Das Erzeugnis nach Anspruch 10, wobei das Positionieren des Erzeugnisses auf der Wunde das kontinuierliche Positionieren des Erzeugnisses auf der Wunde für mehr als 3 Tage umfasst.

12. Das Erzeugnis nach Anspruch 10, wobei das Positionieren des Erzeugnisses auf der Wunde das kontinuierliche Positionieren des Erzeugnisses auf der Wunde für mehr als 4 Tage umfasst.

13. Das Erzeugnis nach Anspruch 10, wobei das Positionieren des Erzeugnisses auf der Wunde das kontinuierliche Positionieren des Erzeugnisses auf der Wunde für mehr als 5 Tage umfasst.

## Revendications

1. Article, comprenant :
un réseau de brins polymères interconnectés ;
dans lequel chacun des brins polymères interconnectés a une hauteur (H1) de 100 à 2000 µm et une largeur moyenne (T) de 10 à 500 µm ;
dans lequel chacun des brins polymères interconnectés possède une première surface adaptée pour entrer en contact avec un site tissulaire ; dans lequel aucun des brins polymères interconnectés ne présente de caractéristique s'étendant à partir de la première surface des brins polymères interconnectés ;
dans lequel au moins l'un des brins polymères interconnectés n'est pas linéaire ; et
une pluralité d'ouvertures entre des brins polymères adjacents interconnectés ;
dans lequel l'article est un article de traitement de plaie par pression négative, et
dans lequel le rapport d'aspect des ouvertures est compris entre plus de 1:1 et 100:1, dans lequel le rapport d'aspect est un rapport d'aspect entre la longueur (L1) et la largeur (W1) des ouvertures, dans lequel la longueur (L1) d'une ouverture est la plus grande distance latérale parallèle à une direction x, et la largeur (W1) d'une ouverture est la plus grande distance parallèle à une direction y.

2. Article selon la revendication 1, dans lequel au moins un des brins polymères interconnectés est linéaire.

3. Article selon l'une quelconque des revendications 1 à 2, dans lequel le réseau comprend une alternance de brins polymères non linéaires et de brins polymères linéaires.

4. Article selon l'une quelconque des revendications 1 à 3, dans lequel le brin polymère non linéaire a une courbe sinusoïdale.

5. Article selon l'une quelconque des revendications 1 à 4, dans lequel la résistance à la traction de l'article parallèle à la direction x est supérieure à 2,23 Mpa.

6. Article selon l'une quelconque des revendications 1 à 5, dans lequel les brins polymères comprennent un polymère élastomère.

7. Article selon la revendication 6, dans lequel le polymère élastomère est choisi parmi les polyoléfines ou les polyuréthanes.

8. Article selon l'une quelconque des revendications 1 à 7, dans lequel essentiellement tous les brins polymères interconnectés sont non linéaires.

9. Système comprenant :
l'article selon l'une quelconque des revendications 1 à 8 ; et
une source de pression réduite reliée fluidiquement à l'ouverture de l'article pour délivrer la pression réduite à travers l'ouverture et jusqu'au site tissulaire.

10. Article selon l'une quelconque des revendications 1 à 8 pour une utilisation dans le positionnement de l'article sur une plaie.

11. Article selon la revendication 10, dans lequel le positionnement de l'article sur la plaie comprend le positionnement continu de l'article sur la plaie pendant plus de 3 jours.

12. Article selon la revendication 10, dans lequel le positionnement de l'article sur la plaie comprend le positionnement continu de l'article sur la plaie pendant plus de 4 jours.

13. Article selon la revendication 10, dans lequel le positionnement de l'article sur la plaie comprend le positionnement continu de l'article sur la plaie pendant plus de 5 jours.
